# EUROPEAN PATENT APPLICATION

(11) **EP 4 116 288 A1**
(43) Date of publication of application: **11.01.2023**
(21) Application number: 21184477.4
(22) Date of filing: 08.07.2021
(51) Int. Cl.: C07C 213/00, C07C 213/08, C07C 221/00, C07C 225/16, C07C 217/72, C07C 217/62

(54) **RACEMIZATION OF (S) AND/OR (R)-3-(DIMETHYLAMINO)-1-(3-METHOXYPHENYL)-2-METHYLPROPAN-1- ONE AND ITS MIXTURES**

(71) Applicant: KRKA, d.d., Novo mesto, 8000 Novo mesto (SI)
(72) Inventor: Bezensek, Jure, 8000 Novo mesto (SI); Andolsek, Barbara, 8000 Novo mesto (SI)
(74) Representative: Hoffmann Eitle

(57) **Abstract**

The present invention provides means and methods for synthesizing tapentadol with increased yield. The processes of the present invention involve a step of racemization of (R)-3-(dimethylamino)-1-(3-methoxyphenyl)-2-methylpropan-1-one.

## Description

### Field of the Invention

The present invention relates to the field of organic synthesis. More specifically, the present invention provides means and methods for synthesizing tapentadol.

### Background of the Invention

During the synthesis of Tapentadol and/or its salts, 3-(dimethylamino)-1-(3-methoxyphenyl)-2-methylpropan-1-one (Formula I) is synthesized as a key intermediate. This key intermediate is obtained in the racemic form. The target product tapentadol, on the other hand, is a chiral substance in stereochemically pure form. Therefore, in the next stage of the synthesis, the enantiomers of the key intermediate are separated using diastereoisomeric crystallization. That means that in the first stage of diastereoisomeric crystallization, a salt is formed from the intermediate and an enantiomerically pure acid, and in the second stage of the process, this salt, due to its low solubility, precipitates from the solvent used.

Various enantiomerically pure acids can be used and the main goal to achieve separation of enantiomers is always that during the diastereoisomeric crystallization only the desired diastereoisomeric salt of the intermediate and the selected enantiopure acid should precipitate.

Diastereoisomeric crystallization is the key step in the tapentadol synthesis. It determines the stereochemistry of the final product. Also, the efficiency of resolution in this step determines the enantiomeric purity of the final product.

The diastereoisomeric crystallization yields the desired (S)-enantiomer of the key intermediate. Said intermediate is isolated and used further in the synthesis. Since the undesired (R)-enantiomer stays in the mother liquor, it is discharged as waste.

The synthesis of tapentadol and its intermediates is e.g. disclosed by the following patent applications and patents:
WO 2008/012047 relates to a process for the preparation of (1R,2R)-3-(-dimethylamino-1 ethyl-2-methyl-propyl)-phenol.
EP 2 046 726 B1 relates to an improved process for the preparation of (2R,3R)-3-(3-metoxyphenyl)-N,N,2-trimethylpentanamine which is an intermediate for preparation of the analgesic tapentadol.
WO 2008/012046 relates to an improved process for the preparation of 3-[(1R,2R)-3-(dimethylamino)-1-ethyl-2-methylpropyl]phenol monohydrochloride.
WO 2011/083304 concerns prodrugs of opioid analgesics and pharmaceutical compositions containing such prodrugs. Methods for providing more consistent pain relief by increasing the bioavailability of the opioid analgesic with the aforementioned prodrugs are provided. The invention also provides for decreasing the adverse GI side effects of opioid analgesics.
WO 2008/012283 relates to a process for the preparation of (2R,3R)-3-(3-methoxyphenyl)-N,N,2-trimethylpentanamine which is an intermediate for the preparation of the analgesic tapentadol.
WO 2014/005546 relates to a preparation method of tapentadol hydrochloride and compounds for preparation of the tapentadol hydrochloride. The preparation method comprises steps using a compound in a formula V or a hydrochloride thereof as a raw material for preparation of the tapentadol hydrochloride, and is characterized in that the preparation method of the hydrochloride of the compound in the formula V comprises the step that in solvent, a hydrochloride of a compound in a formula IV reacts with a hydrogenolysis reagent in the presence of a catalytic agent. The application asserts that chiral column separation is not needed, a removal condition of protecting group allyl is mild, yield is high, and industrialization production is benefited.
WO 2012/001571 has the object of providing a process for the synthesis of tapentadol, both as free base and in hydrochloride form, which comprises a step of alkylation of a ketone. It is asserted that the process yields the product with high stereoselectivity due to the presence of a benzyl group as a substituent of the amino group. It is stated that this substitution shifts the keto-enol equilibrium towards the desired enantiomer and amplifies the capacity of the stereocenter present in the compound (VII) to orient the nucleophilic addition of the organometallic compound at the carbonyl towards the desired stereoisomer. The application claims that this substitution thus allows obtaining a considerable increase of the yields in this step, and consequently allows significantly increasing the overall yield of the entire tapentadol synthesis process. A further object of this application is the provision of the tapentadol free base in solid form by means of the described process. Still another object of this application is the provision of crystalline forms I and II of the tapentadol free base and of the mixture of the crystalline forms I and II of the tapentadol free base.

During tapentadol and/or its salts synthesis intermediate 3-(dimethylamino)-1-(3-methoxyphenyl)-2-methylpropan-1-one (Formula I) is synthesized using dimethyl amine hydrochloride and paraformaldehyde in an appropriate solvent.

Since no asymmetric conditions are used during the synthesis of the above-mentioned key intermediate, it is formed and optionally isolated in the form of a racemate which means as a mixture of 50 % of (S)-3-(dimethylamino)-1-(3-methoxyphenyl)-2-methylpropan-1-one or S isomer and 50 % of (R)-3-(dimethylamino)-1-(3-methoxyphenyl)-2-methylpropan-1-one or R isomer. The structure of this compound is shown by Formula I below.

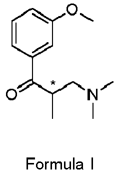

In the next step of the synthesis, resolution of the enantiomers of racemic 3-(dimethylamino)-1-(3-methoxyphenyl)-2-methylpropan-1-one can be achieved by means of diastereoisomeric crystallization using various chiral enantiomerically pure acids. During this approach, only the desired S enantiomer precipitates as a diastereoisomeric salt. After isolation, and optionally neutralization, it is used further in the synthesis of tapentadol and/or its salts. This means that a yield of diastereoisomeric crystallization can theoretically be at maximum 50 %, since only 50 % of S-enantiomer is present in racemic mixture. After the isolation of precipitated S-diastereoisomer salt the opposite R-enantiomer stays in the mother liquor and is discarded as waste. This problem is illustrated by the following reaction Scheme I.

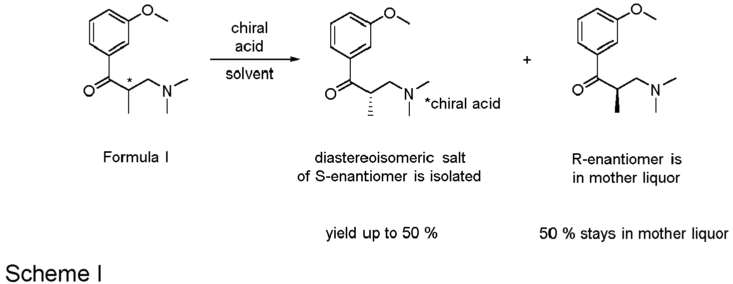

### Summary of the Invention

The present invention has the objective of increasing the overall yield of the state-of-the-art process of preparing tapentatdol. A further objective is to make the process more environmentally friendly. Further objectives become apparent from the detailed description and examples provided hereinbelow.

The above objectives are accomplished by the process of the present invention, which involves a step of reusing (i.e. regenerating) the R enantiomer of the key intermediate to form racemic 3-(dimethylamino)-1-(3-methoxyphenyl)-2-methylpropan-1-one, which can be used again in diastereoisomeric crystallization (Scheme II).

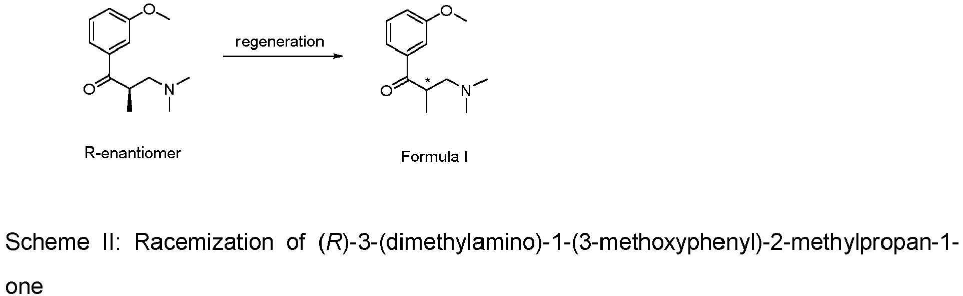

More specifically, the objectives of the present application are accomplished by the processes of the appended claims.

### Detailed Description of the Invention

### Definitions

In the context of the present application, and unless expressly indicated otherwise or the context dictates otherwise, references to a specific **stereoisomer,** e.g. **enantiomer,** are intended to refer not only to the stereomerically pure stereoisomer (enantiomer), but also to mixtures in which the stereoisomer (enantiomer) is enriched. For example, a reference to the (S)-enantiomer of a substance is intended to characterize the enantiomerically pure (S)-form and additionally all mixtures of the (S)-enantiomer with the (R)-enantiomer with the (S)-enantiomer being enriched, i.e. all mixtures in which a greater amount of (S)-enantiomer is present compared to the amount of (R)-enantiomer, which concretely means all mixtures with a mixing ratio of (S)-enantiomer to (R)-enantiomer from 100:0 down to 52:48, thus excluding the racemic mixture. Likewise, a reference to the (R)-enantiomer of a substance is intended to characterize the enantiomerically pure (R)-form and additionally all mixtures with a mixing ratio of (R)-enantiomer to (S)-enantiomer from 100:0 down to 52:48, thereby again excluding the racemic mixture.

The mixing ratio (and thus enantiomeric excess) can be determined by subjecting the mixture to chiral HPLC followed by determination of the relative areas of the respective peaks for the two enantiomers in the chromatogram. Suitable chiral HPLC columns are Chiralpak AD-H or Chiralcel OJ-H; suitable solvents are hexane/ethanol in different ratios with addition of suitable amine (diethylamine).

Enantiomeric excess (purity) was assessed by high pressure liquid chromatography (HPLC, isocratic elution):

| | |
|---|---|
| Column: | Chiralcel OJ-H; (250 x 4.6) mm; 5 µm particles |
| Mobile phase: | hexane : ethanol: diethylamine = 700 : 300 : 1 (V/V/V) |
| Column temperature: | 20°C |
| Flow: | 0.5 ml/min |
| Detection: | UV, wavelength 248 nm |
| Injection volume: | 5 µl |

In the context of the present application, and unless expressly indicated otherwise or the context dictates otherwise, references to **racemic mixtures** are meant to characterize not only a 50:50 mixture of the (S)-enantiomer and the (R)-enantiomer, but also mixtures of the (S) and (R) enantiomers, wherein one of the two enantiomers is in slight excess. That is, a reference to a racemic mixture is meant to characterize all mixtures of (S)-enantiomer to (R)-enantiomer having a mixing ratio of from 48:52 to 52:48.

When characterizing mixtures of stereoisomers (enantiomers), the components of such mixtures all have the same molecular weight. Hence, mixing ratios may be calculated using substance amounts either indicated as number of molecules (i.e. in moles) or on a weight basis (i.e. in grams), but it is of course essential that all amount indications are provided in the same unit.

**Enantiomeric excess,** or "ee", is defined as ee% = (R - S) / (R + S) * 100%, wherein R represents the molar amount of the (R)-enantiomer and S represents the molar amount of the (S)-enantiomer.

The **pKa** of an acid HA ⇆ H⁺ + A⁻ is defined as -log [c(H⁺)*c(A⁻)/c(HA)] and it can be determined by half-titrating the acid and determining the pH. At that stage of half-titration, c(A⁻) = c(HA) and, according to the Henderson-Hasselbalch equation, pKa = pH. In case of multivalent acids, the pKa value of the most acidic proton is to be considered.

The **pKb** of a base B + H₂O ⇆ BH⁺ + OH⁻ is defined as -log [c(BH⁺)*c(OH⁻)/c(B)]. In aqueous solution, pKb of base B is related to the pKa of the corresponding acid BH via the following equation: pKa + pKb = 14.

The classifications of the strengths of acids and bases according to the present invention is based on literature data or experiments at room temperature, e.g. 25°C, with water as the solvent. This applies even for the embodiments of the present invention in which the racemization reaction of the present invention is carried out at a different temperature and/or in a non-aqueous solvent.

In the context of the present application, and unless expressly indicated otherwise or the context dictates otherwise, a **moderate strength acid** is meant to be an acid that has a pKa (or, in case of multivalent acids capable of releasing multiple protons, its lowest pKa) in the range of from 0 to 6, preferably in the range of from 1 to 5.

Any acid stronger than the moderate strength acid, as defined herein, is to be understood as a **strong acid.** Hence, depending on the selected definition for the moderate strength acid, the strong acid has a pKa below 0 or below 1, as the case may be.

In the context of the present application, and unless expressly indicated otherwise or the context dictates otherwise, a **strong base** is meant to be a base B for which the conjugate acid BH⁺ has a pKa of 12 or higher, preferably 13 or higher (corresponding to a pKb value of 2 or less, preferably 1 or less). There is no particular upper limit to the basicity of the strong base catalysts that can be used in the present invention. According to one embodiment, superbases with a pKa greater 15 or even greater 20 are included. According to another embodiment, superbases with a pKa greater 20 are not included.

Any base weaker than a strong base but having a basicity higher than or equal to aniline (having a pKb of 9.4) is considered to be a **moderate strength base.** Hence, pKb values for moderate strength bases are typically in the range of from 2 to 9.4, preferably in the range of from 3 to 8 (corresponding to pKa values in the range of from 4.6 to 12 and preferably 6 to 11).

In the context of the present application, and unless expressly indicated otherwise or the context dictates otherwise, references to the **(R)-enantiomer** are intended to refer to (*R*)-3-(dimethylamino)-1-(3-methoxyphenyl)-2-methylpropan-1-one while references to the **(S)-enantiomer** are intended to refer to (S)-3-(dimethylamino)-1-(3-methoxyphenyl)-2-methylpropan-1-one.

In the context of the present application, and unless expressly indicated otherwise or the context dictates otherwise, all indications apply to a temperature of 22°C. References to room temperature are meant in one embodiment to specify any temperature in the range of 20°C to 25°C. In another embodiment, room temperature is meant to be 22°C.

In the context of the present invention, the term "about", when used in connection with a numerical value, is intended to specify a possible variation of ±20% and preferably ±10% around the specified value.

### Overview

The key intermediate of the tapentadol synthesis, 3-(dimethylamino)-1-(3-methoxyphenyl)-2-methylpropan-1-one (including both of its enantiomers), possesses a carbonyl moiety and a chiral centre in an alpha position relative to the carbonyl group. The chiral carbon atom is a tetrahedral tertiary carbon (sp³ hybridization), which possesses one hydrogen atom. Without wishing to be bound to theory, it is speculated that the racemization of the present invention is associated with a sequence of deprotonation-protonation steps or protonation-deprotonation steps involving the formation of an achiral intermediate planar sp² hybridization state.

The present invention thus accomplishes the above objectives by means of a step of converting (R)-3-(dimethylamino)-1-(3-methoxyphenyl)-2-methylpropan-1-one to a racemic mixture of (R) and (S) forms of 3-(dimethylamino)-1-(3-methoxyphenyl)-2-methylpropan-1-one, as shown in the above Scheme II. This racemization reaction step is advantageously combined with the step of converting the (S)-enantiomer into a diastereomeric salt and isolating the same.

The isolated (S)-enantiomer may optionally be purified. It may then be further reacted to yield tapentatol.

With the procedure of the invention for racemisation of the undesired enantiomer (R)-3-(dimethylamino)-1-(3-methoxyphenyl)-2-methylpropan-1-one, it is possible to convert/racemise it back to the racemic 3-(dimethylamino)-1-(3-methoxyphenyl)-2-methylpropan-1-one, which may then be used for diastereoisomeric crystallisation. This means that using the procedure of the invention for racemisation has the beneficial effect of increasing the overall yield of the diastereoisomeric crystallisation, which means that the overall yield of the tapentadol synthesis is also increased. The procedure of the invention further allows to avoid discarding the undesired (R)-enantiomer, which means that waste can be reduced, and the process can be made more environmentally friendly.

### The Racemisation Reaction

The racemization reaction of the present invention advantageously comprises the following steps 1, 2 and 4 and optionally one or more of the steps 3, 5 and/or 6:
1) Dissolving or suspending the key intermediate 3-(dimethylamino)-1-(3-methoxyphenyl)-2-methylpropan-1-one in the form of its (R) enantiomer in a solvent or a combination of solvents.
2) Adding an acid or base catalyst. This can be done in solid form or dissolved or suspended in a solvent or combination of solvents.
3) Increasing the temperature of the reaction mixture.
4) Maintaining the reaction mixture at the reaction temperature for a period of time sufficient to accomplish racemization.
5) Cooling the reaction mixture.
6) Isolating the racemic product.

The relative order of steps 1 and 2 may also be reversed, i.e. acid or base catalyst may be dissolved or dispersed before adding the key intermediate in (R) form. Alternatively, steps 1 and 2 may be carried out simultaneously. Step 3 is to be carried out before Step 4 and may be carried out before, simultaneously or after Step 1 and/or Step 2. Steps 5 and 6 are to be performed in the specified order after Step 4. Alternatively, the two Steps 5 and 6 may be carried out simultaneously after completion of Step 4.

### Catalyst

The racemization reaction of the present invention is catalyzed by the presence of an acid or a base.

Suitable acids can be selected from the groups of organic acids and inorganic acids. Preferably the acid is a moderate strength acid. Typical moderate strength acids are carboxylic acids of the type R-COOH wherein R is selected from the group consisting of C₁₋₁₂-alkyl groups, C₂₋₁₂-alkenyl groups, C₂₋₁₂-alkynyl groups, C₄₋₈-cycloalkyl groups, C₆₋₁₄-aryl groups, heteroaryl groups with 5 to 10 ring members and 1 to 4 heteroatoms independently selected from N, O and S, each of these groups may carry 0, 1, 2, 3 or 4 substituents selected from OH, COOH, OCOR', COOR', F, CI, Br, I, NO₂, and CN, with R' representing a group selected from C₁₋₄-alkyl, NH₂, NHR" and phenyl and R" representing a group selected from C₁₋₄-alkyl, and phenyl. The open chain alkyl, alkenyl or alkynyl groups may be linear or branched. Cyclic groups may be monocyclic, bicyclic or tricyclic (provided the number of ring atoms permits bicyclic or even tricyclic groups). Preferred moderate strength acids include acetic acid (AcOH), dibenzoyl tartaric acid (DBTA, wherein each of the racemic form and both L- and R-enantiomers may be used), mandelic acid and mixtures thereof.

Strong acids may also be used. They include HCI, HBr, para-toluenesulfonic acid (p-TsOH), Methanesulfonic acid (MsOH), HNO₃ or HClO₄. The use of H₂SO₄ is not preferred. Hence, according to embodiments of the present invention, H₂SO₄ is not among the acids that may be used as a catalyst. For strong acids, it is more important to select appropriate racemization reaction conditions so that the reaction proceeds at an acceptable rate. This typically involves the use of higher temperatures, as described in more detail below.

However, having energy consumption and associated cost in mind, it is more preferred to use a moderate strength acid and to select a lower reaction temperature, as specified hereinbelow specifically for the moderate strength acid catalysts.

Suitable base catalysts may be selected from the groups of organic and anorganic bases. Preferably the base catalyst is a strong base such as sodium hydroxide, potassium hydroxide, lithium hydroxide, potassium tert-butoxide (KOtBu), sodium butoxide, lithium butoxide, and mixtures thereof.

In other embodiments, a moderate strength base may be used, for instance ammonia or an amine. Typical amines include primary amines such as C₁₋₄-alkyl amine or aniline, secondary amines such as di(C₁₋₄-alkyl)amine or tertiary amines such as tri(C₁₋₄-alkyl)amine. Alternatively, it is possible to use mono- or bicyclic aromatic amines such as pyridine. For moderate strength bases, it is more important to select suitable reaction conditions such as higher temperatures, as described in more detail below.

The use of a strong base is however preferred.

The acid or base catalyst is typically added as a pure substance or in a solvent in dissolved or suspended form. If it is added in a solvent, the same solvents may be used as described hereinbelow for the racemization reaction.

In another embodiment, the acid or base catalyst may be immobilized on a solid support. This could be, for instance, the acidic (protonated) form of a cation ion exchange resin as an acid catalyst, and the hydroxyl form of an anion ion exchange resin as a base catalyst. Other solid catalysts showing acidic or basic properties (e.g. acidic or basic zeolites) may of course be used, too.

### Concentration of catalyst

The concentration of the catalyst is not particularly restricted. Generally, any amount of catalyst can be used, wherein the choice of a suitable amount should be based on the consideration that a satisfactory reaction rate can be accomplished with the chosen amount of catalyst. There is no need to employ any greater amount. However, using amounts greater than necessary can be helpful if there is a desire to further accelerate the racemization reaction and/or if it is intended to isolate the racemate by precipitation upon salt formation with the catalyst. In this latter embodiment, a catalyst amount of up to about 100 mol% may be employed. Even amounts of catalyst higher than 100 mol% such as up to 150 mol% or even up to 200 mol% may be used. However, when considering the use high amounts of catalyst, it is advisable to ensure that an amount of catalyst is used that can be removed after the racemization without undue efforts and/or that does not interfere with the subsequent diastereomeric crystallization.

Typical catalyst concentrations for use with moderate strength acids or strong bases are 1 to 50 mol %, preferably 5 to 20 mol% (with mol% indications being calculated as the number of moles of catalyst divided by the total number of moles of starting material, i.e. such that a number of moles of catalyst equal to the number of moles of starting material would correspond to a catalyst concentration of 100 mol%). If a strong acid or a moderate strength base is used, the catalyst amount is typically 10 to 70 mol %, preferably 15 to 60 mol %.

When using an acid or base catalyst immobilized on a solid support and the process is carried out as a batch process, the above-indicated catalyst concentrations may be applied to the number of moles of acidic or basic groups that are immobilized. If the process is carried out in a continuous manner, suitable catalyst amounts depend on further factors like the space velocity of the starting material passing the immobilized catalyst, geometry of the reaction chamber, etc., so that a suitable amount of catalyst is advantageously determined in an iterative manner by step-wise increasing or decreasing the amount until a satisfactory degree of racemization is accomplished for the intended process parameters. Of course, a similar iterative optimization procedure can also be applied to batch processes of the invention in order to optimize the catalyst concentration.

### Solvent

Suitable solvents can be selected from water and organic solvents and mixtures thereof. Preferred solvents are selected from the group of alcohols, ketones, esters, acetonitrile, water and mixtures thereof, more preferably from the group consisting of acetone, methanol, ethanol, isopropanol, dichloromethane and mixtures of thereof.

In an embodiment, when using a moderate strength acid, and especially DBTA, it is preferred to use a solvent selected from ethanol, methanol, acetone or a mixture of acetone and methanol in a volume ratio of 2:1 to 1:2 and preferably 1:1. It is even more preferred to use a moderate strength acid, and especially DBTA, together with a preferred solvent or solvent mixture selected from the list given in the preceding sentence and adjust reaction temperature to a temperature within the range of 25°C to 55°C, preferably 30°C to 50°C and more preferably 35°C to 45°C, as specified hereinbelow for moderate strength acids.

In another embodiment, when using a moderate strength acid, and especially acetic acid, any solvent selected from ethanol, acetone, acetonitrile, toluene, methanol, and mixtures thereof is preferred. It is even more preferred to use a moderate strength acid, and especially acetic acid, together with a preferred solvent or solvent mixture selected from the list given in the preceding sentence and adjust reaction temperature to a temperature within the range of 25°C to 55°C, preferably 30°C to 50°C and more preferably 35°C to 45°C, as specified hereinbelow for moderate strength acids.

When using a strong acid such as HCI, it is preferred to use ethanol as the solvent. It is even more preferred to use HCI and ethanol and adjust reaction temperature to a temperature in the range of from 45 to 65°C and more preferably 48 to 62°C.

When using a strong base such as NaOH or KOtBu, it is preferred to use ethanol, acetone, acetonitrile, toluene or mixtures thereof as the solvent. It is even more preferred to use a strong base such as NaOH or KOtBu, together with a preferred solvent or solvent mixture selected from the list given in the preceding sentence, and adjust reaction temperature to a temperature within the range of 25°C to 55°C, preferably 30°C to 50°C and more preferably 35°C to 45°C, as specified hereinbelow for strong bases.

### Starting material

The starting material is (R)-3-(dimethylamino)-1-(3-methoxyphenyl)-2-methylpropan-1-one contained in the mother liquor, as obtained by the diastereomeric crystallization of (S)-3-(dimethylamino)-1-(3-methoxyphenyl)-2-methylpropan-1-one from the corresponding racemate after separation of the precipitated diastereomeric salt.

According to one embodiment, it is possible to use the mother liquor after separation of the precipitated diastereomeric salt but without further isolation of the starting material. According to another embodiment, the starting material may be isolated from the mother liquor. Isolation may be accomplished by addition of an acid to form a salt with low solubility. Isolation may also be accomplished by evaporation of volatile components, followed by one or more extractions into suitable solvents such as a mixture of toluene and water, and finally evaporation of said suitable solvents. Yet another option is to use the mother liquor after separation of the precipitated diastereomeric salt, without further isolation of the (R)-3-(dimethylamino)-1-(3-methoxyphenyl)-2-methylpropan-1-one but with removal of the chiral acid such as L-DBTA that is used for diastereomeric crystallization of (S)-3-(dimethylamino)-1-(3-methoxyphenyl)-2-methylpropan-1-one. If the chiral acid is not removed prior to racemization, it is advantageous and therefore preferred to remove the chiral acid from the racemized product. If the chiral acid is not removed prior to racemization, the remaining amount of chiral acid is to be considered when determining the amount acid or base that needs to be added in order to accomplish a concentration of catalyst in accordance with the relative amount indication provided above for the catalyst. For example, if there is a small amount of L-DBTA remaining and if it is intended to use NaOH as the base catalyst, it is necessary to add an amount of NaOH necessary for accomplishing the desired catalyst concentration plus and additional amount of NaOH which corresponds to the amount of remaining L-DBTA (to neutralize this acid). The purity of the starting material is not particularly restricted.

Of course, if the mother liquor is used without prior isolation of the starting material, there is no need for dissolving the starting material although the addition of further solvent is possible.

The optical purity of the starting material is not particularly restricted as it depends primarily on the reaction conditions selected for the diastereomeric crystallization. Typically, the optical purity of the (R)-3-(dimethylamino)-1-(3-methoxyphenyl)-2-methylpropan-1-one starting material is in the range of from 65%ee or more, or of 80%ee or more, or even 90%ee or more.

In principle, it is also possible to carry out the racemization process of the present invention using the opposite enantiomer, i.e. (S)-3-(dimethylamino)-1-(3-methoxyphenyl)-2-methylpropan-1-one, as the starting material. This aspect, however, provides no use in the context of the tapentadol synthesis and it is therefore a less preferred embodiment of the present invention.

### Concentration of starting material

The concentration of the starting material is not particularly limited. For reasons of efficiency, it is advantageous to select a concentration not less than 10 mg/mL, preferably not less than 50 mg/mL. The upper limit of the concentration of the starting material is typically 500 mg/mL, preferably 250 mg/mL, more preferably 200 mg/mL.

### Dissolving

The starting material may be dissolved in the selected solvent or solvent mixture by any suitable means. Typically, the starting material is added to the solvent (mixture) or the solvent (mixture) is added to the starting material, followed by stirring. This can be done at room temperature, but any other temperature, in which the selected solvent (mixture) is liquid, may also be used. Increasing the temperature may be advantageous as it increases the dissolution rate. According to one embodiment, the dissolution of the starting material is carried out at the reaction temperature, as described hereinbelow. This means that the solvent is heated to the reaction temperature either before or after adding the starting material.

### Suspending

The starting material need not be completely dissolved in the solvent (mixture). According to one embodiment, the reaction mixture is a heterogenous system in which a part or all of the starting material (and/or the catalyst) is present in undissolved solid or oily form. In this embodiment, it is advantageous to subject the reaction mixture to stirring. If the starting material is in solid form, e.g. after precipitation with an acid, it may also be advantageous to subject the starting material (and/or catalyst) to grinding prior to the reaction in order to reduce particle sizes and thus increase surface area.

### Reaction temperature

The reaction temperature is not particularly limited, provided the racemization reaction proceeds at an acceptable rate. It is typically within the temperature range of from 15°C to 85°C, preferably 25°C to 75°C, more preferably 35°C to 65°C and especially 40°C to 60°C. Of course, the maximum temperature is further limited by the boiling point of the selected solvent. Hence, if it is desired to use a reaction temperature of, for instance, 75°C, it is advisable to select a solvent having a boiling point of 75°C or higher.

If a strong acid is used as a catalyst, it is advisable to select a higher racemization reaction temperature within the range of 50°C to 85°C, preferably within the range of 55°C to 80°C and more preferably within the range of 60°C to 75°C.

If a moderate strength acid is used as a catalyst, the racemization reaction temperature is not particularly restricted and any temperature within the above-identified ranges may be used. Having regard to cost and environmental considerations, it may be advantageous to select lower temperatures as for the moderate strength acid embodiment. For instance, temperatures within the range of 25°C to 55°C, preferably 30°C to 50°C and more preferably 35°C to 45°C may be selected when using moderate strength acids as catalysts.

If a strong base is used as a catalyst, the racemization reaction temperature is not particularly restricted and any temperature within the above-identified ranges may be used. Having regard to cost and environmental considerations, it may be advantageous to select lower temperatures as for the strong base embodiment. For instance, temperatures within the range of 25°C to 55°C, preferably 30°C to 50°C and more preferably 35°C to 45°C may be selected when using strong base catalysts.

If a moderate strength base is used as a catalyst, it is advisable to select a higher racemization reaction temperature within the range of 50°C to 85°C, preferably within the range of 55°C to 80°C and more preferably within the range of 60°C to 75°C. It is furthermore preferred to increase the pressure during the reaction to a pressure above atmospheric pressure, e.g. a pressure in the range of from 0.12 MPa to 1.00 MPa, preferably 0.15 MPa to 0.50 MPa. Ideally, this is combined with a rather high catalyst concentration of typically 10 to 70 mol %, preferably 15 to 60 mol %, as mentioned above.

The step (3) of increasing the temperature includes in particular an increase of the temperature from room temperature to the reaction temperature, as specified above. The heating rate is not particularly restricted.

The step (5) of cooling includes in particular the cooling of the reaction mixture after completion of the racemization reaction from the reaction temperature, as specified above, to room temperature. Alternatively, cooling may also be performed such that the end temperature is higher or lower than room temperature such as a temperature in the range of from 0°C to 20°C or a temperature in the range of from 25°C to 30°C. The cooling range is not particularly restricted.

### Duration of reaction

The duration of the reaction is not particularly restricted. Of course, it is appropriate to continue with the reaction until an acceptable degree of racemization is accomplished. The duration of the reaction is thus linked to the selected reaction conditions and the desired degree of racemization.

For reasons of practicability, it is preferred that the duration of the reaction is 48h or less, more preferably 24h or less, even more preferably 20h or less and most preferably 12h or less. The lower limit of the reaction duration is also determined by the chosen reaction conditions and it is typically 10 min or more, preferably 30 min or more, such as 1h or more or, more typically, 2h or more.

When using a moderate strength acid, preferred durations of the reaction are in the range of from 2h to 30h, more preferably 3h to 24h, even more preferably 4h to 20h. In case of using a strong base, preferred durations of the reaction are in the range of from 10 min to 20h, more preferably 30 min to 12h, even more preferably 1h to 8h.

In one embodiment, the course of the reaction may be monitored, for instance by performing repeated measurements of enantiomeric excess at regular time intervals. The reaction may be stopped at a point in time when the drawbacks of additional time and energy needed for accomplishing further racemization exceed the benefits of increased yields.

In view of the above, the racemization may be continued until a racemic mixture (52:48 or lower, down to 50:50) is accomplished. Alternatively, the racemization reaction may already be stopped e.g. at a ratio of enantiomers of 52.5:47.5 or lower, 54:46 or lower, 55:45 or lower, 56:44 or lower, 58:42 or lower, or even 60:40 or lower, wherein the term "or lower" in this context refers to any ratio down to 50:50. In other words, the racemized product may have 4%ee or less, 5%ee or less, 8%ee or less, 10%ee or less, 12%ee or less, 16%ee or less or even 20%ee or less. Of course, smaller degrees of enantiomeric excess are preferred in view of the above-mentioned objective of increasing the overall yield in the tapentadol synthesis. However, as noted hereinabove, the aim of striking a reasonable balance between yield and reaction time and energy may justify terminating the reaction already earlier, at higher degrees of enantiomeric excess.

If a chiral acid such as L-DBTA is used as the catalyst for the racemization, it is also possible that the desired (S)-enantiomer salt, e.g. (S)-enantiomer L-DBTA salt, precipitates during the racemization reaction. In this case, the ratio of (R):(S)-enantiomers *in solution* may be within the ranges specified above (or even higher), but when additionally taking the precipitated (S)-enantiomer salt, e.g. (S)-enantiomer L-DBTA salt, into account, the conversion may progress even beyond the 50:50 ratio. Hence, according to one embodiment of the invention, a chiral acid such as L-DBTA is used as the catalyst for racemization and reaction conditions are selected (e.g. reaction temperature of 30 °C to 50 °C and preferably 35 °C to 45 °C), which permit precipitation of the diastereomeric salt of the catalyst with the (S)-enantiomer. In one aspect of this embodiment, the precipitated diastereomeric salt may be removed by suitable solid-liquid separation methods like filtration or centrifugation and the remaining liquid phase may then be subjected to diastereomeric crystallization. In an alternative aspect of this embodiment, after racemization, further diastereomeric crystallization may be performed by lowering the temperature to a temperature in the range of 0 °C to 25 °C and preferably 0°C to 10°C without prior removal of the diastereomeric salt precipitated already during racemization. In this aspect, the entire amount of precipitated diastereomeric salt may then be isolated using a suitable solid-liquid separation method like filtration or centrifugation.

### Stirring

The reaction mixture may optionally be stirred during the steps of dissolution, addition of catalyst and also during the reaction step. Stirring is particularly advantageous whenever there is a heterogenous mixture, e.g. when starting material and/or catalyst is not completely dissolved, and/or whenever there is a concentration gradient or temperature gradient within the reaction vessel.

The stirring equipment is not particularly restricted. Suitable stirrers include for instance magnetic stirrers, static stirrers, turbines, impellers and the like. Stirring speed is also not particularly restricted.

### Batch size

The reaction of the invention may be performed at any scale, including laboratory scale (up to batches of 1 kg of starting material), pilot plant scale (typical batch sizes of 3 to 30) and industrial scale (typical batch sizes of 30 to 300) as well as any intermediate scale.

The reaction of the present invention is typically carried out in a batch-wise manner. However, it is also possible to carry out the reaction of the present invention in a continuous manner using e.g. an immobilized catalyst.

### Isolation and further use of the racemic product

The racemic product can be isolated by evaporating the solvent. The employed acid or base catalyst may be removed by neutralization and/or extraction with an immiscible solvent. Another possibility is to isolate a racemate salt formed with an acid, such as the acid used as a catalyst in the process. In this embodiment, it is required to add a sufficient quantity of the acid to enable effective precipitation. This means that further acid should be added such that the total acid amount is at least 100 mol% of the racemate.

After its isolation, the racemic product may be dissolved in a solvent and subjected to diastereomeric crystallization to isolate the desired (S)-enantiomer. Alternatively, it is possible to perform diastereomeric crystallization to isolate the desired (S)-enantiomer using the solution of the racemic product as it is obtained from the racemization reaction of the present invention, optionally after removal of the catalyst by neutralization and/or extraction with an immiscible solvent.

Diastereomeric crystallization of the racemized product can be performed in the same manner as described below in relation to the preparation of the starting material.

### Method or preparing Tapentadol

(S)-3-(dimethylamino)-1-(3-methoxyphenyl)-2-methylpropan-1-one can be further converted to tapentadol according to following scheme:

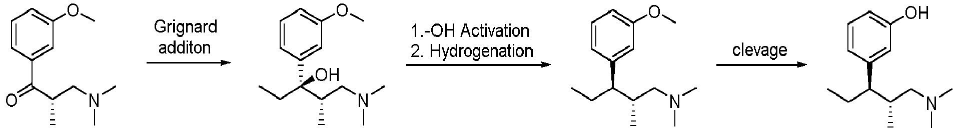

### Method of preparing starting material, diastereomeric crystallization

The (R)-enantiomer of 3-(dimethylamino)-1-(3-methoxyphenyl)-2-methylpropan-1-one is obtained from the racemic 3-(dimethylamino)-1-(3-methoxyphenyl)-2-methylpropan-1-one by diastereomeric crystallization, wherein the (S)-enantiomer is removed by precipitation of a diastereomeric salt formed with a suitable chiral acid (sometimes also referred to as a chiral auxiliary agent). Suitable chiral acids are in principle not particularly limited. However, L-dibenzoyl tartaric acid (L-DBTA), has proven to be particularly suitable. Hence, the use of this acid is preferred.

The process of diastereomeric crystallization is carried out in a solvent or in a solvent mixture. Suitable solvents may be selected from ketones (such as acetone, methyl isobutyl ketone (MIBK)), alcohols (such as methanol (MeOH), ethanol (EtOH), isopropyl alcohol (IPA)), and hydrocarbons (such as toluene) and mixtures thereof. It is preferable to use the same solvent or same mixture of solvents for the diastereomeric crystallization and for the subsequent racemization reaction, especially if the mother liquor is to be used as a starting material for the racemization reaction.

The process of diastereomeric crystallization comprises the steps of dissolving the racemic 3-(dimethylamino)-1-(3-methoxyphenyl)-2-methylpropan-1-one in the selected solvent (mixture), followed by addition of the selected chiral acid, precipitation of the diastereomeric salt of (S)-3-(dimethylamino)-1-(3-methoxyphenyl)-2-methylpropan-1-one with the chiral acid and subsequent isolation of the precipitated diastereomeric salt. Isolation may be accomplished by for instance by filtration, decantation or centrifugation.

Suitable conditions for carrying out diastereomeric crystallization are described at page 19 of WO 2008/012047 under items 1.a and 1.b. It is possible to rely on these conditions when performing diastereomeric crystallization in the context of the present invention.

Racemic 3-(dimethylamino)-1-(3-methoxyphenyl)-2-methylpropan-1-one can be obtained as follows: 1-(3-methoxyphenyl)propan-1-one, dimethylamine hydrochloride, paraformaldehyde are dissolved in isopropanol and concentrated hydrochloric acid is added. The reaction mixture is heated up to reflux temperature and mixed until complete conversion to 3-(dimethylamino)-1-(3-methoxyphenyl)-2-methylpropan-1-one. After evaporation of volatile components, the product is purified with extractions into toluene and water. At the end toluene and other volatile components are evaporated to yield racemic 3-(dimethylamino)-1-(3-methoxyphenyl)-2-methylpropan-1-one.

### Examples

### Example 1

(S)-3-(dimethylamino)-1-(3-methoxyphenyl)-2-methylpropan-1-one (0,50 g, R 2,45 %, S 97,55 %, ee 95,10 %) was dissolved in ethanol (5 mL) and selected acid (99-100 % acetic acid 0,135 mL or L-dibenzoyl tartaric acid monohydrate (L-DBTA) 886 mg) was added at room temperature and reaction mixture heated up to 40 °C. The reaction mixture was sampled after 2 h and 17 h. Results of racemization in ethanol with the tested acids are summarized in the table 1.

**Table 1**

| | 2h | | | 17 h | | |
|---|---|---|---|---|---|---|
| | R | S | ee | R | S | ee |
| AcOH | 35,96 | 64,04 | 28,08 | 49,30 | 50,70 | 1,4 |
| L-DBTA | 1,21 | 98,79 | 97,58 | 55,91 | 44,09 | -11,82* |

| | | | | | | |
|---|---|---|---|---|---|---|
| *precipitation | | | | | | |

### Example 2

(S)-3-(dimethylamino)-1-(3-methoxyphenyl)-2-methylpropan-1-one (0,50 g, R 2,45 %, S 97,55 %, ee 95,10 %) was dissolved in acetone (5 mL) and 99-100 % acetic acid 0,135 mL was added at room temperature and reaction mixture heated up to 40 °C for 19 h. HPLC ee analyses showed that racemisation occurred (R-isomer 46.06 area % and S-isomer 53.94 area %).

### Example 3

(S)-3-(dimethylamino)-1-(3-methoxyphenyl)-2-methylpropan-1-one (0,50 g, R 2,45 %, S 97,55 %, ee 95,10 %) was dissolved in acetonitrile (5 mL) and 99-100 % acetic acid 0,135 mL was added at room temperature and reaction mixture heated up to 40 °C for 19 h. HPLC ee analyses showed that racemisation occurred (R-isomer 49.74 area % and S-isomer 50.26 area %).

### Example 4

(S)-3-(dimethylamino)-1-(3-methoxyphenyl)-2-methylpropan-1-one (0,50 g, R 2,45 %, S 97,55 %, ee 95,10 %) was dissolved in toluene (5 mL) and acetic acid 0,135 mL was added at room temperature and reaction mixture heated up to 40 °C for 19 h. HPLC ee analyses showed that racemisation occurred (R-isomer 39.85 area % and S- isomer 60.15 area %).

### Example 5

(S)-3-(dimethylamino)-1-(3-methoxyphenyl)-2-methylpropan-1-one (0,5 g) (S-enantiomer 98.82 area %) was dissolved in mixture of acetone (5 mL) and methanol (5 mL) and stirred at 40 °C for 24 h. HPLC analyses showed that no racemisation occurred. L-dibenzoyl tartaric acid (25 mg) was added to the reaction mixture and reaction was heated further for 24 h at 40 °C. HPLC ee analyses showed that racemisation occurred. (R-isomer 40.77 area % and S- isomer 59.23 area %).

### Example 6

(S)-3-(dimethylamino)-1-(3-methoxyphenyl)-2-methylpropan-1-one (1 g) (S-enantiomer min 98 area %) was dissolved in ethanol (1,88 mL) and 8 M HCI in EtOH (0,62 mL) was added to the reaction mixture at room temperature. The reaction mixture was heated to 50 °C and stirred further for 20 h. HPLC ee analyses showed there is racemate present in reaction mixture. (R-isomer 49.93 area % and S- isomer 50.47 area %).

### Example 7

Mixture of (S)-3-(dimethylamino)-1-(3-methoxyphenyl)-2-methylpropan-1-one (82.78 area %) and (R)-3-(dimethylamino)-1-(3-methoxyphenyl)-2-methylpropan-1-one (17.22 area %) obtained from mother liquor after resolution with L-DBTA (41,68 g) was dissolved in Ethanol (78,4 mL) and 8 M HCI in EtOH (25,84 mL) was added to the reaction mixture at room temperature. The reaction mixture was heated to 60 °C and stirred further for 20 h. HPLC ee analyses showed there is racemate present in reaction mixture. (R-isomer 50.03 area % and S- isomer 49.97 area %).

### Example 8

(S)-3-(dimethylamino)-1-(3-methoxyphenyl)-2-methylpropan-1-one (0,50 g, R 2,45 %, S 97,55 %, ee 95,10 %) was dissolved in ethanol (5 mL) and selected base (NaOH 94,2 mg or KOtBu 264 mg) was added at room temperature and reaction mixture heated up to 40 °C. The reaction mixture was sampled after 2 h and 17 h. Results of racemization in ethanol with various bases are summarized in the table.

**Table 2**

| | 2h | | | 17 h | | |
|---|---|---|---|---|---|---|
| | R | S | ee | R | S | ee |
| NaOH | 48,35 | 51,65 | 3,3 | 49,22 | 50,78 | 1,56 |
| KOtBu | 48,70 | 51,30 | 2,6 | 49,28 | 50,71 | 1,43 |

### Example 9

(S)-3-(dimethylamino)-1-(3-methoxyphenyl)-2-methylpropan-1-one (0,50 g, R 2,45 %, S 97,55 %, ee 95,10 %) was dissolved in acetone (5 mL) and selected base (NaOH 94,2 mg or KOtBu 264 mg) was added at room temperature and reaction mixture heated up to 40 °C. The reaction mixture was sampled after 2 h and 19 h. Results of racemization in ethanol with various bases are summarized in the table.

**Table 3**

| | 2h | | | 17 h | | |
|---|---|---|---|---|---|---|
| | R | S | ee | R | S | ee |
| NaOH | 49,82 | 50,18 | 0,36 | 50,17 | 49,83 | -0,34 |
| KOtBu | 49,87 | 50,13 | 0,26 | 47,53 | 52,47 | 4,94 |

### Example 10

(S)-3-(dimethylamino)-1-(3-methoxyphenyl)-2-methylpropan-1-one (0,50 g, R 2,45 %, S 97,55 %, ee 95,10 %) was dissolved in acetonitrile (5 mL) and selected base (NaOH 94,2 mg or KOtBu 264 mg) was added at room temperature and reaction mixture heated up to 40 °C. The reaction mixture was sampled after 3 h and 19 h. Results of racemization in ethanol with various bases are summarized in the table.

**Table 4**

| | 3h | | | 19 h | | |
|---|---|---|---|---|---|---|
| | R | S | ee | R | S | ee |
| NaOH | 39,04 | 60,96 | 21,92 | 49,83 | 50,17 | 0,34 |
| KOtBu | 49,16 | 50,84 | 1,68 | 48,31 | 51,05 | 2,74 |

## Claims

1. A process of preparing racemic 3-(dimethylamino)-1-(3-methoxyphenyl)-2-methylpropan-1-one, which comprises the steps of
a. Dissolving or suspending as a starting material the (S) or (R)-form of 3-(dimethylamino)-1-(3-methoxyphenyl)-2-methylpropan-1-one in a solvent or providing a solution or dispersion of the (S) or (R)-form of 3-(dimethylamino)-1-(3-methoxyphenyl)-2-methylpropan-1-one in a solvent;
b. Adding a catalyst;
c. Optionally increasing the temperature;
d. Maintaining the reaction mixture at the reaction temperature for a period of time sufficient for the racemization reaction to proceed;
e. Optionally cooling the reaction mixture; and
f. Optionally isolating the reaction product, which is partially or completely racemized 3-(dimethylamino)-1-(3-methoxyphenyl)-2-methylpropan-1-one;
wherein step (d) is carried out after steps (a), (b) and, if present, (c), wherein steps (e) and (f), if present, are carried out after step (d) and wherein the relative order of steps is not further restricted;
wherein the catalyst is selected from the group consisting of acids, mixtures of acids, bases and mixtures of bases; and
wherein the solvent is selected from the group consisting of water, organic solvents and mixtures thereof.

2. The process according to claim 1, wherein the starting material has an enantiomeric excess 65%ee or more, or of 80%ee or more, or even 90%ee or more.

3. The process according to claim 1 or 2, wherein the reaction product has an enantiomeric excess of 20%ee or less, or 10%ee or less, or even 5%ee or less.

4. The process according to any one of claims 1 to 3, wherein the temperature in step (c) is increased to a temperature in the range of from 35°C to 65°C or even 40°C to 60°C, followed by step (d) and cooling in step (e).

5. The process according to any one of claims 1 to 4, wherein the catalyst is a moderate strength acid such as acetic acid, dibenzoyl tartaric acid, mandelic acid and mixtures thereof.

6. The process according to any one of claims 1 to 4, wherein the catalyst is a strong base such as sodium hydroxide, potassium hydroxide, lithium hydroxide, potassium tert-butoxide, sodium butoxide, lithium butoxide and mixtures thereof.

7. The process according to any one of claims 1 to 6, wherein the solvent is selected from the group consisting of alcohols, ketones, esters, acetonitrile, water and mixtures thereof, in particular from the group consisting of acetone, methanol, ethanol, isopropanol, dichloromethane and mixtures of thereof.

8. The process of any one of claims 1 to 7, wherein the starting material used in step (a) has a molar ratio of the (R)-form to (S)-form of 3-(dimethylamino)-1-(3-methoxyphenyl)-2-methylpropan-1-one in the range of from 60:40 to 100:0 or even 70:30 to 100:0.

9. A method of preparing tapentadol, the method comprising the steps of
(i) providing a racemic mixture of 3-(dimethylamino)-1-(3-methoxyphenyl)-2-methylpropan-1-one;
(ii) subjecting the racemic mixture of step (i) to diastereomeric crystallization using a chiral auxiliary agent to isolate the (S)-enantiomer of 3-(dimethylamino)-1-(3-methoxyphenyl)-2-methylpropan-1-one;
(iii) converting the remaining (R)-enantiomer of 3-(dimethylamino)-1-(3-methoxyphenyl)-2-methylpropan-1-one of step (ii) or (iv) into a racemic mixture of 3-(dimethylamino)-1-(3-methoxyphenyl)-2-methylpropan-1-one by means of the process according to any one of the preceding claims;
(iv) subjecting the racemic mixture of step (iii) to diastereomeric crystallization using a chiral auxiliary agent to isolate the (S)-enantiomer of 3-(dimethylamino)-1-(3-methoxyphenyl)-2-methylpropan-1-one;
(v) optionally repeating steps (iii) and (iv) one or more times; and
(vi) one or more steps of converting (S)-3-(dimethylamino)-1-(3-methoxyphenyl)-2-methylpropan-1-one obtained in steps (ii) and (iv) into tapentadol.

10. The method according to claim 9, wherein each of the steps (iii) and (iv) is carried out 2, 3 or 4 times, wherein the remaining (R) enantiomer of 3-(dimethylamino)-1-(3-methoxyphenyl)-2-methylpropan-1-one of step (ii) or (iv) is used as a starting material in step (iii).

11. The method according to claim 9 or 10, wherein step (vi) includes the following reaction steps (vi-1), (vi-2) and (vi-3):
(vi-1) conversion of (S)-3-(dimethylamino)-1-(3-methoxyphenyl)-2-methylpropan-1-one into (2S,3R)-1-(dimethylamino)-3-hydroxy-3-(3-methoxyphenyl)-2-methylpentan;
(vi-2) conversion of (2S,3R)-1-(dimethylamino)-3-hydroxy-3-(3-methoxyphenyl)-2-methylpentan into (2R,3R)-1-(dimethylamino)-3-(3-methoxyphenyl)-2-methylpentan;
(vi-3) conversion of (2R,3R)-1-(dimethylamino)-3-(3-methoxyphenyl)-2-methylpentan into (1R,2R)-3-(3-dimethylamino-1-ethyl-2-methyl-propyl)-phenol (tapentadol).

12. The method of any one of claims 9, 10 or 11, wherein the conversion of step (iii) is in accordance with claim 1 and optionally further fulfills one or more of the features of claims 2 to 7.

13. The process according to any of claims 1 to 8 or the method of claim 9, 10, 11 or 12, wherein 3-(dimethylamino)-1-(3-methoxyphenyl)-2-methylpropan-1-one is obtained by means of a preceding step of synthesis from 1-(3-methoxyphenyl)propan-1-one.
